# EUROPEAN PATENT APPLICATION

(11) **EP 3 444 602 A1**
(43) Date of publication of application: **20.02.2019**
(21) Application number: 18189570.7
(22) Date of filing: 17.08.2018
(51) Int. Cl.: G01N 27/327

(54) **MEASURING METHOD AND MEASURING APPARATUS**

(30) Priority: 17.08.2017 JP 2017157662; 09.08.2018 JP 2018150637
(71) Applicant: ARKRAY, Inc., Kyoto-shi, Kyoto 601-8045 (JP)
(72) Inventor: KANEDA, Hisashi, Kyoto-shi, Kyoto 602-0008 (JP); NAKATANI, Ayano, Kyoto-shi, Kyoto 602-0008 (JP)
(74) Representative: Dehns

(57) **Abstract**

A measuring method includes applying a first signal, which is a direct current signal, having a first value to a sample in a flow passage (9) using a first electrode (6) and a fourth electrode (7) which is different from the first electrode (6), second electrode (4), and third electrode (5), measuring a first electric response value, applying a second signal having a second value higher than the first value to the sample using the second electrode (4) and third electrode (5), measuring a second electric response value, and correcting a value obtained from the first electric response value based on the second electric response value.

## Description

### FIELD

The present disclosure relates to a measuring method and a measuring apparatus.

### BACKGROUND

Conventionally, a technique is known, in which a value of concentration of glucose (glucose value) and a hematocrit value (Hct value) in blood, which is an example of a sample are measured, and the glucose value is corrected by using the Hct value. For example, a method is known as the method for measuring the glucose value and the Hct value, in which the Hct value is measured by using a counter electrode and a working electrode provided with a mediator and the glucose value is measured by using a counter electrode and a working electrode provided with a reagent.

Alternatively, a method is known, in which the Hct value is measured by applying an AC voltage to a working electrode and a counter electrode provided with a reagent respectively, and the glucose value is measured by applying a direct current voltage. Further, a method is known, in which a common working electrode is used for the measurement of the glucose value and the measurement of the Hct value, and the both measurements are performed by applying a direct current voltage. Further alternatively, the following method is known. That is, a first electric response value is measured with respect to a first signal inputted into a first electrode pair capable of making contact with a sample. A second signal, which is inputted into a second electrode pair capable of making contact with the sample, is used, the second signal having a value which changes from a first level to a second level, and the second signal thereafter maintaining the second level. A second electric response with respect to the second signal is measured as a peak value of a response signal with respect to the change of the second signal. A value, which indicates the amount of a target substance of the sample as obtained from the first electric response, is corrected based on the peak value of the response signal.

For further information, see Japanese Patent No. 4611208, Japanese Patent Application Laid-Open No. 2016-510124 (PCT), Japanese Patent Application Laid-Open No. 2005-147990, and Japanese Patent Application Laid-Open No. 2014-232102.

At least preferred embodiments of the present invention provide a measuring method and a measuring apparatus which make it possible to accurately measure a target substance contained in a biological sample by using a new technique.

### SUMMARY

A first aspect of the invention provides a measuring method for measuring a target substance contained in a biological sample by using an analysis tool including a flow passage for the sample and a plurality of electrodes arranged in the flow passage. The plurality of electrodes includes the first electrode which is provided with a reagent including an enzyme, a second electrode and a third electrode each of which is not provided with the reagent, and the fourth electrode which is different from the first electrode, the second electrode, and the third electrode.

The measuring method includes:
applying a first signal, which is a direct current signal, having a first value to the sample disposed in the flow passage by using the first electrode as a working electrode and using the fourth electrode as a counter electrode;
measuring a first electric response value of the sample to the first signal;
applying continuously for a given time a second signal, which is a direct current signal, having a second value higher than the first value to the sample in the flow passage by using the second electrode and the third electrode as the working electrode and the counter electrode;
measuring a second electric response value of the sample within the given time to the second signal, the second electric response value indicating amount of electric charge generated by electrolysis of water in the sample; and
correcting a value obtained from the first electric response value based on the second electric response value.

Another aspect of the invention provides a measuring method for measuring a target substance contained in a biological sample by using an analysis tool including a flow passage for the sample and a plurality of electrodes arranged in the flow passage. The plurality of electrodes including the first electrode which is provided with a reagent including an enzyme, and the second electrode and the third electrode each of which is not provided with the reagent.

The measuring method includes:
applying a first signal, which is a direct current signal, having a first value to the sample in the flow passage by using the first electrode as a working electrode and using one of the second electrode and the third electrode as a counter electrode;
measuring a first electric response value of the sample to the first signal;
applying continuously for a given time a second signal, which is a direct current signal, having a second value higher than the first value to the sample in the flow passage by using the second electrode and the third electrode as the working electrode and the counter electrode;
measuring a second electric response value of the sample within the given time to the second signal, the second electric response value indicating amount of electric charge generated by electrolysis of water in the sample; and
correcting a value obtained from the first electric response value based on the second electric response value.

The advantages of at least preferred embodiments of the invention will be realized and attained by means of the elements and combinations particularly pointed out in the claims.

It is to be understood that both the foregoing general description and the following detailed description are exemplary and explanatory and are not restrictive of the invention.

### BRIEF DESCRIPTION OF THE DRAWINGS

Certain preferred embodiments will now be described by way of example only and with reference to the accompanying figures in which:
FIG. 1A illustrates a top view illustrating an analysis tool (biosensor having four electrodes) according to an embodiment, and FIG. 1B illustrates a side view illustrating the biosensor illustrated in FIG. 1A;
FIG. 2A illustrates a top view illustrating an analysis tool (biosensor having three electrodes) according to an embodiment, and FIG. 2B illustrates a side view illustrating the biosensor illustrated in FIG. 2A;
FIG. 3A and FIG. 3B illustrate exemplary arrangements of measuring apparatuses according to embodiments;
FIG. 4 illustrates a flow chart illustrating an exemplary measuring method based on the use of the biosensor and the measuring apparatus according to the embodiment;
FIG. 5A illustrates the correlation (corresponding relationship) between the second electric response value and the Hct value (linearity of the Hct calibration curve) provided when the glucose value is constant;
FIG. 5B illustrates the time-dependent change (time course) of the second electric response value provided when the glucose value is constant;
FIG. 5C illustrates the time-dependent change of the first electric response value provided when the glucose value is constant;
FIG. 6A illustrates the time-dependent change of the second electric response value provided when the Hct value is constant, and FIG. 6B illustrates the time-dependent change of the first electric response value provided when the Hct value is constant.

### DESCRIPTION OF EMBODIMENTS

An explanation will be made below about the measuring method and the measuring apparatus according to embodiments. In the embodiments, an explanation will be made about a measuring method for measuring a target substance contained in a biological sample by using an analysis tool including a flow passage for the sample and a plurality of electrodes arranged in the flow passage. The measuring method includes a first measuring method and a second measuring method. In the first measuring method, the plurality of electrodes includes the first electrode which is provided with a reagent including an enzyme, a second electrode and a third electrode each of which is not provided with the reagent, and the fourth electrode which is different from the first electrode, the second electrode, and the third electrode. In the second measuring method, the plurality of electrodes includes the first electrode which is provided with a reagent including an enzyme and the second electrode and the third electrode each of which is not provided with the reagent.

The first measuring method comprises a step of applying a first signal, which is a direct current signal, having a first value to the sample in the flow passage by using the first electrode as a working electrode and using the fourth electrode as a counter electrode; a step of measuring a first electric response value of the sample to the first signal; a step of applying continuously for a given time a second signal, which is a direct current signal, having a second value higher than the first value to the sample in the flow passage by using the second electrode and the third electrode of the plurality of electrodes as the working electrode and the counter electrode; a step of measuring a second electric response value of the sample within the given time to the second signal, the second electric response value indicating amount of electric charge generated by electrolysis of water in the sample; and a step of correcting a value obtained from the first electric response value based on the second electric response value.

The second measuring method comprises a step of applying a first signal, which is a direct current signal, having a first value to the sample in the flow passage by using a first electrode as a working electrode and using one of the second electrode and the third electrode as a counter electrode; a step of measuring a first electric response value of the sample to the first signal; a step of applying continuously for a given time a second signal, which is a direct current signal, having a second value higher than the first value to the sample in the flow passage by using the second electrode and the third electrode as the working electrode and the counter electrode; a step of measuring a second electric response value of the sample within the given time to the second signal, the second electric response value indicating amount of electric charge generated by electrolysis of water in the sample; and a step of correcting a value obtained from the first electric response value based on the second electric response value. In the first and second measuring methods, the corrected value is dealt with as the measured value of the target substance contained in the sample.

The analysis tool, which is applied to the first and second measuring methods, is, for example, a biosensor. In the first and second measuring methods, the sample is, for example, a biological sample. However, the sample may include a sample other than the biological sample. The biological sample is a liquid sample which is, for example, blood, interstitial fluid, or urine. The target substance contained in the sample is, for example, a glucose value (blood sugar value) or a lactate value (lactic acid value), and the like. The reagent may contain an enzyme and a mediator.

In the first and second measuring methods, it is preferable that the sample is blood. Further, it is preferable that the target substance is glucose. It is preferable that the second electric response value of the sample is a value which indicates hematocrit. Further, it is preferable that the first electric response value of the sample is a value which indicates glucose before being corrected by the value which indicates hematocrit.

In the first and second measuring methods, the measurement of the first electric response value and the measurement of the second electric response value are preferably performed at different timings. As for the order of the measurement, any one may be performed earlier. In the present invention, the first signal has the first value and the second signal has the second value different from the first value. Each of the first signal and the second signal is, for example, a voltage whose direction does not change with time, namely a direct current voltage. The first signal may be called the first voltage and the second signal may be called the second voltage. The first value and the second value differ depending on the type of the mediator, the enzyme, and the electrode material. However, as the absolute condition, "first value < second value" is fulfilled (the second value is larger than the first value).

A lower limit value of direct current voltage applied to electrodes, in order to cause electrolysis of water, is about 1.0 V in view of the voltage at which electrolysis of water occurs. When the direct current voltage is 1.0 V or more, an electric response value is not affected by the target substance in the sample. On the other hand, bubbles are generated in the sample as the applied voltage increases, and the electrical response value becomes a value influenced by bubbles. Therefore, an upper limit of the applied voltage is about 7.0 V at which bubbles are not generated. For example, in a case of using ruthenium as a material of electrodes (using a ruthenium electrode), it is preferable that the lower limit of the applied voltage is 1.5 V and the upper limit of the applied voltage is 5.0 V. When ruthenium is used as the electrode material then the direct current voltage value as the first signal (example of the first value) is, for example, about 0.5 V (which is, for example, below a value of direct current voltage at which electrolysis of water occurs), and the direct current voltage value as the second signal (example of the second value) is, for example, about 2.5 V (which is, for example, above a value of direct current voltage at which electrolysis of water occurs). However, as mentioned above, the upper limit of the second value is about 7 V at which bubbles are not produced. In the present invention, the second value of the direct current voltage, which is applied as the second signal, is, for example, 2.5 V, preferably from 1.8 V to 7 V, and more preferably from 2 V to 5 V. The given time to perform continuous application of the second signal is, for example, 3 seconds, preferably from 2 seconds to 5 seconds, and more preferably from 2.5 seconds to 4 seconds . The first value of the direct current voltage as the first signal is the value which is smaller than the second value. The first value is, for example, 500 mV, preferably from 50 mV to 1 V, and more preferably 100 mV to 750 mV. The above mentioned ranges of the first and second values are an example in a case where the material of electrodes is ruthenium. A range of an appropriate applied voltage varies depending on a type of enzyme and substrate, namely salt.

In the first and second measuring methods, the second signal, which is not less than the given value, is applied to the sample for not less than the given time. The second electric response value is measured at the point in time at which the application continues for the given time after the start thereof (i.e. at the point in time following the application of the second signal for the given time) . This is based on the following measurement principle. The second signal having the second value (for example, direct current voltage) is applied to the sample in the flow passage of the analysis tool (electrode for measuring the second electric response value). In this procedure, the second electric response value, which is provided in a period of about 1 second after the start of the application, exhibits an attenuation curve depending on the concentration of the target substance (for example, glucose value) contained in the sample. On the contrary, when about 2 seconds elapse after the start of the application, the second electric response value exhibits a constant value depending on the Hct value of the sample irrelevant to (independent of) the concentration of the target substance. Such a phenomenon occurs as follows.

That is, the charge transfer advances in an order of "target substance -> reagent -> electrode" in accordance with the application of the second signal. Further, the charge transfer occurs in an order of "H₂O -> electrode" in accordance with the electrolysis of water (H₂O) contained in the sample. However, the force, with which the electric charge transfers from H₂O to the electrode, is stronger than the force with which the electric charge transfers from the target substance via the reagent to the electrode. Note that when the reagent contains the enzyme and the mediator, the charge transfer advances in an order of "target substance -> reagent (enzyme -> mediator) -> electrode".

On this account, the second electric response value, which is caused by the charge transfer from the target substance, is observed in a certain degree of period (about 1 second) after the start of the application of the second signal. However, the charge transfer from H₂O to the electrode is greater than the charge transfer from the reagent to the electrode after the elapse of the given time (about 2 seconds). The reagent is saturated while holding the electric charge, and the charge transfer does not occur from the reagent to the electrode. That is, the second electric response value, which is provided in response to the application of the second signal, is substantially the value which indicates the electric charge amount generated by the electrolysis of H₂O, after the elapse of about 2 seconds (given time) after the start of the application of the second signal. In other words, the second electric response value is a value indicating amount of electric charge generated by electrolysis of water in the sample, and the second electric response value is obtainable by continuously applying the second signal for a given time. Note that when the reagent contains the enzyme and the mediator, the disappearance of the charge transfer from the reagent to the electrode means any one of the disappearance of the charge transfer from the enzyme to the mediator and the disappearance of the charge transfer from the mediator to the electrode and the both.

In this context, the hematocrit is the volume of the tangible component (blood cell or hemocyte) contained in blood. Therefore, the high Hct value means the small amount of H₂O (blood plasma) subjected to the electrolysis. Therefore, the higher the Hct value is, the lower the second electric response value is, the second electric response value being provided (measured) at the point in time at which the application continues for the given time (i.e. at the point in time following the application of the second signal for the given time) . In other words, the second electric response value, which is provided (measured) at the point in time at which the application continues for the given time (i.e. at the point in time following the application of the second signal for the given time), is proportional to the Hct value in accordance with a negative function. Accordingly, if the calibration curve (for example, a table to indicate the correlation) is prepared beforehand between the second electric response value and the Hct value, it is possible to calculate the Hct value from the second electric response value provided (measured) at the point in time at which the application continues for the given time (i.e. at the point in time following the application of the second signal for the given time) . The second electric response value and the Hct value as described above can be used to correct the target substance.

According to the foregoing description, it is possible to understand the following fact. That is, the second electric response value, which exhibits the correct Hct value as compared with the second electric response value provided before the elapse of the given time, can be obtained by measuring the second electric response value provided at the point in time at which the application of the second signal continues for the given time after the start thereof (i.e. at the point in time following the application of the second signal for the given time). That is, it is possible to perform the highly accurate measurement of the Hct value. As a result, it is possible to raise the accuracy of the correction of the target substance. It is possible to perform the measurement of the target substance in which the accuracy is improved. Further, the electrochemical reaction, which is caused by the application of the second signal, is stabilized at the point in time at which the application continues for the given time as compared with the point in time which is provided before the elapse of the given time. Therefore, the Hct value having the high accuracy can be obtained from the second electric response current value, as compared with the Hct value which is determined from the second electric response current value provided before the elapse of the given time.

Further, the analysis tool, which is applied to the first and second measuring methods, includes the plurality of electrodes. In the first measuring method, the plurality of electrodes include the first electrode which is provided with the reagent including the enzyme, the second electrode and the third electrode which are not provided with the reagent respectively, and the fourth electrode which is different from the first electrode, the second electrode, and the third electrode. The reagent is provided on the electrode, for example, by fixing or immobilizing the solid reagent on the electrode.

In the measurement of the first electric response value in the first measuring method, the first electrode is used as the working electrode, and the fourth electrode is used as the counter electrode. In the measurement of the second electric response value, the second electrode and the third electrode are used as the working electrode and the counter electrode. In this way, the electrodes, on which the reagent is not provided, are used as the working electrode and the counter electrode to be used for the measurement of the second electric response value. The reagent may be either provided or not provided on the fourth electrode. However, when the reagent is provided, the accuracy of the first electric response value is improved.

In the second measuring method, the plurality of electrodes include at least the first electrode which is provided with the reagent including the enzyme, and the second electrode and the third electrode which are not provided with the reagent respectively. In the measurement of the first electric response value in the second measuring method, the first electrode is used as the working electrode, and any one of the second electrode and the third electrode is used as the counter electrode. On the other hand, in the measurement of the second electric response value, the second electrode and the third electrode are used as the working electrode and the counter electrode. In this way, also in the second measuring method, the electrodes, on which the reagent is not provided, are used as the working electrode and the counter electrode to be used for the measurement of the second electric response value.

In the first and second measuring methods, when the second electric response value is measured, the electrodes, on which the reagent is not provided, are used as the working electrode and the counter electrode. Accordingly, it is possible to obtain the following advantage. That is, in the production step of producing the analysis tool, for example, a liquid reagent is dripped onto the electrode, or a paste-form reagent is printed thereon, followed by being dried. Thus, the reagent is fixed or immobilized on the electrode. In this procedure, there is a possibility that the situation of solidification of the reagent may be varied or dispersed among individuals of the analysis tool, and the second electric response value may be varied or dispersed among the individuals. In the first measuring method and the second measuring method, the electrodes, on which the reagent is not provided, are used as the working electrode and the counter electrode. Accordingly, it is possible to suppress the scattering or dispersion of the second electric response value among the individuals.

Note that in the second measuring method, it is preferable that one of the second electrode and the third electrode is used as the working electrode for the measurement of the second electric response value, and the other is used as the common counter electrode for both of the measurement of the first electric response value and the measurement of the second electric response value. However, one of the second electrode and the third electrode used as the working electrode for the measurement of the second electric response value may be used as the counter electrode for the measurement of the first electric response value.

Further, in the first and second measuring methods, two or more electrodes may be used as the working electrode and the counter electrode in the measurement of the first electric response value and the measurement of the second electric response value. Further, the plurality of electrodes may include an electrode which is used as the reference electrode and an electrode which is used for the measurement of any measurement item other than the measurement of the first electric response value and the second electric response value.

Here, a state that "reagent is provided on an electrode" includes a state that the regent is in contact with the electrode and is established or installed on the electrode, a state that the regent is fixed or immobilized on the electrode, a state that the regent is put, loaded, mounted, or disposed on the electrode, and the like. However, there is a case where although the reagent is not provided on the working electrode and the counter electrode, the reagent is provided between the working electrode and the counter electrode. The working electrode and the counter electrode in this case are included in "two electrodes used to measure a first electric response value". A state that "the reagent is provided between the working electrode and the counter electrode" means, for example, a state that the reagent is in contact with a substrate between the working electrode and the counter electrode and the reagent is established or installed on the substrate between the working electrode and the counter electrode. The state that "the reagent is provided between a working electrode and a counter electrode" includes, for example, a state that the reagent is in contact with a spacer and/or a cover and the reagent is established or installed on the spacer and/or the cover. Each of the spacer and the cover forms a flow passage between the working electrode and the counter electrode. This state includes, for example, a state that although there is no established or installed position of the reagent on a substrate, there is the reagent between the working electrode and the counter electrode in a planar view of the flow passage. In other words, a state that "the reagent is provided between a working electrode and a counter electrode" means that the established or installed reagent is provided in a state that the reagent diffuses (moves) to the measurement environment in a vicinity of the working electrode due to the influence of the sample introduced into the flow passage.

Further, with respect to "two electrodes used to measure a second electric response value", the reagent is not provided on both of the two electrodes. Moreover, with respect to two electrodes using measurement of the second electric response value (for example, a working electrode and a counter electrode), in a case where a reagent is not provided on both of a working electrode and a counter electrode, but the reagent is provided between the working electrode and the counter electrode, the working electrode and the counter electrode are not included in the "two electrodes used to measure a second electric response value". That is, in the measurement of the second electric response value, it is required that the reagent is not provided on or in not only the working electrode and the counter electrode but also all measurement environment of the second electric response value.

An explanation will be made below with reference to the drawings about the measuring method and the measuring apparatus according to the embodiments. The structure or arrangement of the embodiments explained below is referred to by way of example. The present disclosure is not limited to the structure or arrangement of the embodiments.

In the following embodiments, an explanation will be made about a measuring method and a measuring apparatus, as examples of the measuring method and the measuring apparatus, in which a biosensor as an analysis tool is used, the glucose value in blood is measured as a target substance contained in a biological sample, and the glucose value is corrected by using the Hct value of blood (second electric response value). The present disclosure is not limited to the use of a biosensor as an analysis tool, nor to blood as a sample, nor to glucose as the target substance, nor to correcting on the basis of a Hct value.

### <Structure of biosensor>

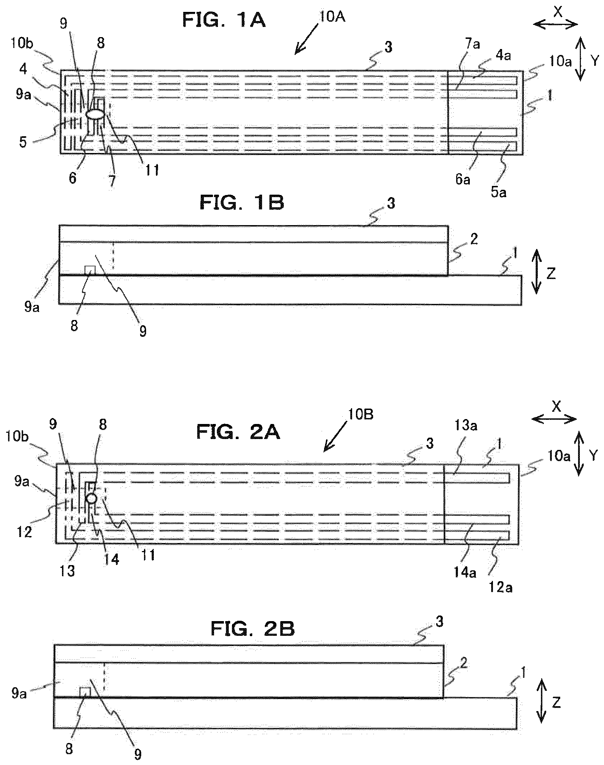

FIGS. 1A and 1B and FIGS. 2A and 2B illustrate exemplary structures of biosensors according to embodiments. FIG. 1A illustrates a top view illustrating an analysis tool (biosensor having four electrodes) according to the embodiment, and FIG. 1B illustrates a side view illustrating the biosensor illustrated in FIG. 1A. FIG. 2A illustrates a top view illustrating an analysis tool (biosensor having three electrodes) according to the embodiment, and FIG. 2B illustrates a side view illustrating the biosensor illustrated in FIG. 2A.

The biosensor 10A may be used in the first measuring method. In FIG. 1A and FIG. 1B, the biosensor 10A (hereinafter referred to as "sensor 10A") has the longitudinal direction (X direction) having one end 10a and the other end 10b and the widthwise direction (Y direction). The sensor 10A is formed by stacking and adhering an insulating substrate 1 (hereinafter referred to as "substrate 1"), a spacer 2, and a cover 3 in the height direction (Z direction).

For example, a synthetic resin (plastic) is used for the substrate 1. As the synthetic resin, for example, it is possible to use various resins such as polyetherimide (PEI), polyethylene terephthalate (PET), polybutylene terephthalate (PBT), polyethylene (PE), polystyrene (PS), polymethacrylate (PMMA), polypropylene (PP), polyimide resin, acrylic resin, epoxy resin, and glass epoxy. Note that any insulating material other than the synthetic resin can be used as the substrate 1. The insulating material includes, for example, paper, glass, ceramic, and biodegradable material, in addition to the synthetic resin. The material, which is the same as that of the substrate 1, can be used for the spacer 2 and the cover 3.

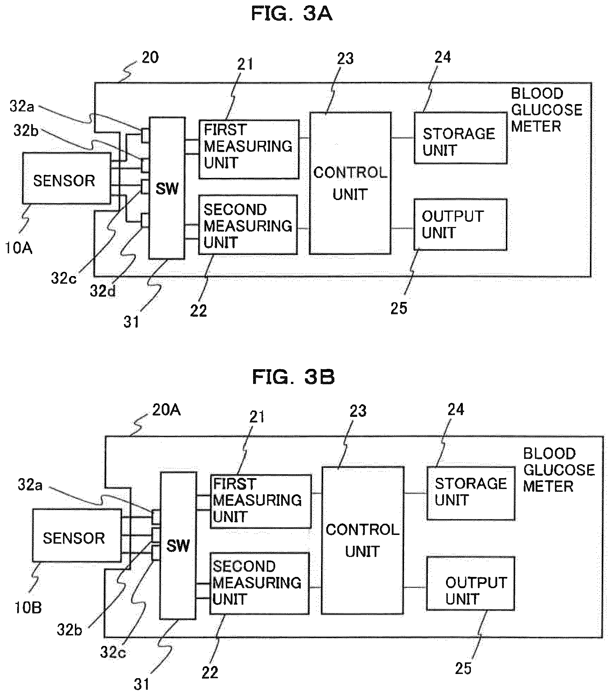

An electrode 4, an electrode 5, an electrode 6 and an electrode 7 are provided on the upper surface of the substrate 1 as one example of a plurality of electrodes. Each of the electrode 4, the electrode 5, the electrode 6 and the electrode 7 has a key-shape (for example, an L-shape) including a portion which extends in the widthwise direction of the sensor 10A and a portion which extends in the longitudinal direction of the sensor 10A. The portion, which extends in the longitudinal direction, is a lead portion 4a, a lead portion 5a, a lead portion 6a and a lead portion 7a. The lead portion 4a, the lead portion 5a, the lead portion 6a and the lead portion 7a, which are disposed on the one end 10a side, are not covered with the spacer 2 and the cover 3. The lead portion 4a, the lead portion 5a, the lead portion 6a and the lead portion 7a are used for the electric connection with connectors included in a blood glucose meter 20 (FIG. 3) .

Each of the electrodes 4, the electrode 5, the electrode 6 and the electrode 7 is formed by using, for example, a metal material or an alloy thereof such as gold (Au), platinum (Pt), silver (Ag), palladium, ruthenium, and nickel or a carbon material such as carbon. For example, each of the electrodes 4, 5, 6 and 7 may be formed as a metal layer having a desired thickness by forming a film of a metal material by means of the physical vapor deposition (PVD, for example, sputtering) or the chemical vapor deposition (CVD). Alternatively, each of the electrodes 4, 5, 6 and 7 may be also formed by printing an ink containing a carbon material on the substrate 1 by means of the screen printing.

The spacer 2 has a cutout portion having a rectangular-shape (recess recessed from the other end 10b toward the one end 10a side) . The cutout portion opens toward the other end 10b side. By laminating or stacking the substrate 1, the spacer 2, and the cover 3, a space, which has an opening 9a formed by the cutout portion of the spacer 2, is formed on the other end 10b side of the sensor 10A. The space is formed by a thickness surface of the cutout portion, an upper surface of the substrate 1 on which the electrodes are provided, and an undersurface (lower surface) of the cover 3. The upper surface of the substrate 1 and the undersurface (lower surface) of the cover face each other into the space (not covered by bonding with the spacer 2) . The space is used as a passage 9 for the sample. An air hole (air vent) 11 is formed through the cover 3. The flow passage 9 is formed so that the sample, which is spotted to the opening 9a, is attracted or drawn (introduced) into the flow passage 9 in accordance with the capillary phenomenon and the sample is moved toward the air hole 11 (biological sample flows through the flow passage 9). Parts of the electrodes 4, 5, 6, and 7 are exposed in the flow passage 9. A reagent 8 is provided (immobilized) on the electrode 6 and the electrode 7. On the other hand, the reagent 8 is not provided on the electrode 4 and the electrode 5.

The reagent 8 contains an enzyme and/or a substrate. In other words, the reagent 8 includes a salt (a salt is included, for example, in an enzyme, a substrate, a mediator, and a reagent). The reagent 8 may further contain a mediator. The enzyme is appropriately selected depending on the type of the sample and the target substance. When the target substance is glucose contained in blood or interstitial fluid, glucose oxidase (GOD) or glucose dehydrogenase (GDH) is applied. The mediator is, for example, ferricyanide, ruthenium complex, p-benzoquinone, p-benzoquinone derivative, phenazine methosulfate, methylene blue, ferrocene, ferrocene derivative, and ruthenium complex. Among them, it is preferable to apply ferricyanide or ruthenium complex, and it is more preferable to apply potassium ferricyanide or ruthenium compounds [Ru(NH₃)₆]cl₃.

The electrode 6 and the electrode 7 are used as the electrode pair to be used for the measurement of the glucose value. For example, the electrode 6 is used as the working electrode, and the electrode 7 is used as the counter electrode or vice versa. The electrode 6 and the electrode 7 are examples of the first electrode and the fourth electrode in the first measuring method. However, when the reagent is provided on the electrode 6 and the electrode 7, then the electrode 6 may be used as the counter electrode, and the electrode 7 may be used as the working electrode. Further, it is also allowable that the reagent is not provided on the electrode (one of the electrode 6 and the electrode 7) which is used as the counter electrode in the measurement of the glucose value.

The electrode 4 and the electrode 5 are used as the electrode pair to be used for the measurement of Hct value. For example, the electrode 4 is used as the working electrode, and the electrode 5 is used as the counter electrode. However, they may be used in an opposite manner. As described above, in the examples illustrated in FIG. 1A and FIG. 1B, the different electrode pairs are used for the measurement of the glucose value and the measurement of the Hct value. The electrode pair having no reagent is used for the measurement of the Hct value. Neither salt component nor mediator is provided on the electrode 4 and the electrode 5 (counter electrode for the measurement of the Hct value).

The biosensor 10B illustrated in FIG. 2A and FIG. 2B (hereinafter referred to as "sensor 10B") is used in the second measuring method. The sensor 10B has a three-electrode structure which is provided with an electrode 12, an electrode 13, and an electrode 14 in place of the electrode 4, the electrode 5, the electrode 6, and the electrode 7 of the sensor 10A. The reagent 8 is fixed or immobilized on the electrode 14. The reagent is not provided on the electrode 12 and the electrode 13, and neither salt component nor mediator is provided thereon.

In the measurement of the glucose value, the electrode 14 is used as the working electrode, and the electrode 13 is used as the counter electrode. On the other hand, in the measurement of the Hct value, the electrode 12 is used as the working electrode, and the electrode 13 is used as the counter electrode. The electrode 14 corresponds to the first electrode in the second measuring method, and the electrode 12 and the electrode 13 correspond to the second electrode and the third electrode in the second measuring method. However, the electrode 12 may be used as the counter electrode when the glucose value is measured.

### <Arrangement of measuring apparatus (blood glucose meter)>

FIG. 3A illustrates a block diagram illustrating an exemplary arrangement of the blood glucose meter 20 as an example of the measuring apparatus. With reference to FIG. 3A, for example, the sensor 10A can be connected to the blood glucose meter 20. The blood glucose meter 20 measures the glucose value by using the connected sensor 10A, and the blood glucose meter 20 corrects the glucose value by using the Hct value.

The blood glucose meter 20 is provided with a first measuring unit 21, a second measuring unit 22, a switch (SW) 31, a control unit 23, a storage unit 24, and an output unit 25. The switch 31 is electrically connected to a plurality of connectors (connector 32a, connector 32b, connector 32c, and connector 32d illustrated in FIG. 3A) . The connector 32a is connected to the lead portion 4a (electrode 4) of the sensor 10A, and the connector 32b is connected to the lead portion 5a (electrode 5) of the sensor 10A. The connector 32c is connected to the lead portion 6a (electrode 6) of the sensor 10A, and the connector 32d is connected to the lead portion 7a (electrode 7) of the sensor 10A.

The switch 31 is the switch which changes the states of electric connection and disconnection thereof between the connector 32a, the connector 32b, the connector 32c, and the connector 32d and the electrode 4, the electrode 5, the electrode 6, and the electrode 7. The control unit 23 is a processor (for example, Central Processing Unit (CPU)) which executes the program stored in the storage unit 24. The storage unit 24 includes a memory which includes a main storage device such as RAM (Random Access Memory) or the like and an auxiliary storage device such as ROM (Read Only Memory), a hard disk or the like. The storage unit 24 stores, for example, the program which is to be executed by the control unit 23 and the data which is to be used when the program is executed. The output unit 25 includes output devices such as a printer, a display device and the like, and communication devices such as a signal connector, a communication interface and the like.

The control unit 23 controls the switch 31 during the measurement of the glucose value so that the working electrode (electrode 6) and the counter electrode (electrode 7) for measuring the glucose value are in the states of being electrically connected to the blood glucose meter 20 and the electrode 4 and the electrode 5 are in the disconnected states. The first measuring unit 21 is configured by circuit (s) and/or a processor and a memory and performs operations below. That is, the first measuring unit 21, according to an instruction of the control unit 23, starts application of the direct current voltage as the first signal between the electrode 6 and the electrode 7. After the direct current voltage is continuously applied for a given time, the first measuring unit 21 measures a response current value corresponding to the glucose value (the first electric response value corresponding to the first signal) as a response current value corresponding to the direct current voltage. For example, the first measuring unit 21 measures, as a first electric response value, a response current value at an end point of the given time. However, a time point or timing to obtain the first electric response value may be a given time point other than the end point of the application of the first signal (a given time point in applying of the first signal).

The control unit 23 controls the switch 31 during the measurement of Hct value so that the working electrode (electrode 4) and the counter electrode (electrode 5) for measuring the Hct value are in the states of being electrically connected to the blood glucose meter 20 and the electrode 6 and the electrode 7 are in the disconnected states. The second measuring unit 22 is configured by circuit(s) and/or a processor and a memory and performs operations below. That is, the second measuring unit 22, according to an instruction of the control unit 23, applies the direct current voltage corresponding to the second signal between the electrode 4 and the electrode 5. After the direct current voltage is continuously applied for a given time, the second measuring unit 22 measures, as the second electric response value, a response current value indicating amount of electric charge generated by electrolysis of water in the sample. However, a time point or timing to obtain the second electric response value may be a given time point other than the end point of the application of the second signal (a given time point in applying of the second signal).

The storage unit 24 stores the calibration curve data for determining the glucose value corresponding to the first electric response value measured by the first measuring unit 21. The control unit 23 converts the first electric response value into the glucose value by using the calibration curve data, and thus the control unit 23 calculates the glucose value. The glucose value is stored in the storage unit 24 in some cases, and/or the glucose value is outputted (for example, displayed) from the output unit 25 in other cases. A calibration curve table may be used to obtain the glucose value instead of the calibration curve data.

Further, the control unit 23 performs a process (Hct correction of the glucose value) in which the glucose value is corrected by using the second electric response value obtained from the second measuring unit 22 or the Hct value obtained from the second electric response value. For example, the storage unit 24 stores the calibration curve data or the calibration curve table which represents correlation or corresponding relationship between the second electric response value and the amount of correction. The control unit 23 determines the amount of correction corresponding to the second electric response value by using the calibration curve data or the calibration curve table. The control unit 23 performs the process for correcting the glucose value, and the control unit 23 calculates the corrected glucose concentration. The corrected glucose value is stored in the storage unit 24 in some cases, and/or the corrected glucose value is outputted (for example, displayed) from the output unit 25 in other cases. Note that when the second electric response value is used to correct the glucose value, it is not necessarily essential to perform the conversion from the second electric response value to the Hct value. The control unit 23 may correction of the second electric response value based on a temperature of measurement environment of the second electric response value using the calibration curve data or a calibration curve table for the correction.

FIG. 3B illustrates a blood glucose meter 20A having a structure corresponding to the sensor 10B having the three-electrode structure. In this case, the connector 32d is omitted, the connector 32a is connected to the lead portion 12a (electrode 12), the connector 32b is connected to the lead portion 13a (electrode 13), and the connector 32c is connected to the lead portion 14a (electrode 14). The switch 31 brings about such a state that the first measuring unit 21 is electrically connected to the electrode 14 and the electrode 13, for example, in accordance with the control effected by the control unit 23 during the measurement of the glucose value.

On the other hand, the switch 31 brings about such a state that the second measuring unit 22 is electrically connected to the electrode 12 and the electrode 13, for example, in accordance with the control effected by the control unit 23 during the measurement of the Hct value. In this way, the switch 31 changes the connection state in relation to the electrodes 12 to 14, the first measuring unit 21, and the second measuring unit 22.

The first measuring unit 21 applies the direct current voltage as the first signal between the electrode 14 as the working electrode and the electrode 13 as the counter electrode to measure the first electric response value. The second measuring unit 22 applies the direct current voltage as the second signal between the electrode 12 as the working electrode and the electrode 13 as the counter electrode to perform the measurement of the second electric response value at the point in time at which the application continues for the given time from the start thereof. Except for the above, the blood glucose meter 20A is constructed in the same manner as the blood glucose meter 20.

### <Exemplary operation>

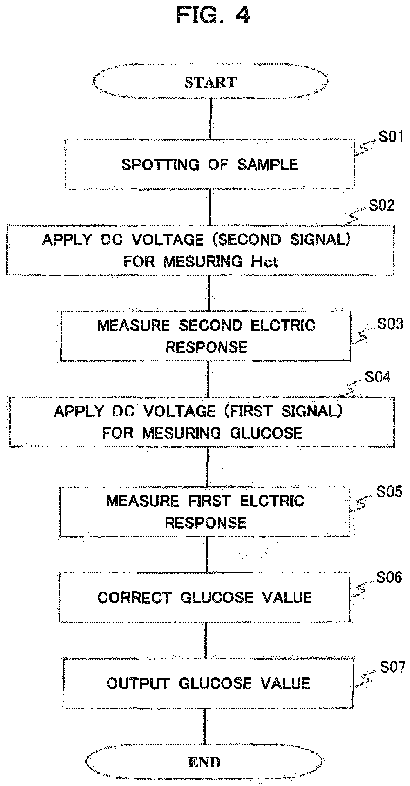

FIG. 4 illustrates a flow chart illustrating an exemplary operation of the blood glucose meter 20. In S01 illustrated in FIG. 4, the spotting of the sample is performed. That is, when the biosensor (for example, sensor 10A) is connected to the blood glucose meter 20, and the opening 9a of the other end 10b of the sensor 10A is brought in contact (subjected to the spotting) with the blood (biological sample) collected from an examinee, then the blood is drawn (attracted) into the flow passage 9 in accordance with the capillary force, and the interior of the flow passage 9 is filled therewith.

The control unit 23 applies the voltage to the electrode before the spotting to observe the applied voltage. The control unit 23 detects the change of the voltage caused by the contact between the blood and the electrode as a result of the spotting, and the control unit 23 detects that the blood is introduced into the flow passage 9. Then, the control unit 23 starts the measurement of the Hct value (S02) .

In S02, the control unit 23 controls the second measuring unit 22 to apply the second signal to the blood. That is, the control unit 23 gives the instruction to the second measuring unit 22 so that the direct current voltage (for example, 2.5 V) corresponding to the second signal is applied to the electrode pair (electrode 4 and electrode 5) for measuring Hct value.

The second measuring unit 22 waits until the application of the direct current voltage corresponding to the second signal continues for the given time (about 2 seconds) after the start thereof, and the second measuring unit 22 measures the second electric response of the blood at the point in time at which the application continues for the given time, namely a response value does not include amount of electric charge based on the reagent and indicates amount of electric charge generated by electrolysis of water in the sample (S03) . For example, the second measuring unit 22 measures the response signal depending on the direct current voltage, and the response signal is subjected to the A/D conversion and sent to the control unit 23.

When the control unit 23 acquires the second electric response of the blood depending on the second signal, the control unit 23 starts the measurement of glucose. That is, the control unit 23 applies the first signal to the blood (S04) . That is, the control unit 23 instructs the first measuring unit 21 that the direct current voltage as the first signal, which is lower than the second signal, is applied to the electrode pair (electrode 6 and electrode 7) for measuring glucose. The first measuring unit 21 applies the direct current voltage in accordance with the instruction.

The first measuring unit 21 measures the first electric response of the blood depending on the first signal (S05). For example, the first measuring unit 21 measures the response current of the first signal. The second measuring unit 22 performs the A/D conversion of the response current to be sent to the control unit 23.

The control unit 23 is operated as the correcting unit to perform the correcting process for the glucose value (S06) . That is, the control unit 23 calculates the value of the correction object component contained in the blood (glucose value) by using the value of the first electric response (response current) acquired in S05, the calibration curve data or the calibration curve table. Further, the control unit 23 calculates the corrected glucose value by using the second electric response value acquired in S03 (or the Hct value corresponding to the second electric response).

The control unit 23 outputs the corrected glucose value (S07) . That is, the control unit 23 stores the glucose value corrected in S06 in the storage unit 24, and the glucose value is displayed on the output unit 25 (display) . The control unit 23 can also transmit the glucose value to any other device via any wired or wireless network by using the output unit 25. Note that even when the sensor 10B and the blood glucose meter 20A are applied, the process, which is the same as or equivalent to that illustrated in FIG. 4, is performed. Therefore, any detailed explanation of the process is omitted. Note that in the exemplary operation illustrated in FIG. 4, the measurement of the Hct value is performed before the measurement of the glucose value. However, the order of the operation may be reversed.

### [Example 1]

In Example 1, an explanation will be made about the measurement of the glucose value and the measurement of the Hct value based on the use of a biosensor in which any reagent is not provided on a working electrode and a counter electrode used for the measurement of the Hct value. As for the biosensor, a biosensor having the structure of the sensor 10A was manufactured, in which the electrodes 4 to 7 were made of ruthenium. The reagent 8 was provided on the electrode 6 and the electrode 7. The formulation and the production method of the reagent 8 are as follows. An enzyme solution was prepared, which contained polyvinyl alcohol (PVA 146,000) 0.8% by weight, ruthenium compound [Ru(NH₃)₆]Cl₃ 1.6% by weight, FAD-GDH 2.7 U, 1-methoxy PMS 0.3% by weight, and ACES buffer (pH 6.5). 0.5 µL of the enzyme solution was dispensed, followed by being dried at 25°C to thereby obtain the reagent 8.

Whole bloods of two people were mixed and used to prepare specimens. Specimens prepared from biological samples, in which the glucose value was 336 mg/dl and the Hct value was 0%, 20%, 42%, and 70% respectively, were prepared. Further, specimens prepared from biological samples, in which the Hct value was 42% and the glucose value was 0, 67 mg/dl, 600 mg/dl, and 800 mg/dl respectively, were prepared. As for the number n of specimens, n = 5 was given.

The evaluation method was carried out in accordance with the following procedure.
1. The Hct values of the specimens were adjusted to 42%.
2. A process, in which a glucose additive solution was added to the samples having the Hct values adjusted to 42% respectively, was carried out to prepare a group of specimens (referred to as "specimen group 1") in which the glucose values were 0 mg/dl, 67 mg/dl, 336 mg/dl, 600 mg/dl, and 800 mg/dl respectively.
3. Further, the adjustment of the Hct value was performed for the specimen in which the glucose value was adjusted to 336 mg/dl to prepare a group of specimens (referred to as "specimen group 2") in which the Hct values were 0%, 20%, 42%, and 70% respectively.
4. The Hct value and the glucose value were measured under the following conditions.

### (1) Measurement of second electric response value (Hct value)

The application of the direct current voltage (2.5 V: corresponding to the second signal having the second value) for measuring Hct value was started 0.5 seconds (0.5 seconds open circuit) after connecting, to the blood glucose meter 20, the electrodes for measuring the Hct value (electrode 4 and electrode 5) of the sensor 10A in which the specimen was introduced into the flow passage. The application of the direct current voltage was continued for 2.5 seconds, and the response current (second electric response value) was measured 2.5 seconds after the start of the application (3 seconds after the start of the measurement).

### (2) Measurement of first electric response value (glucose value)

The application was stopped 2.5 seconds after the start of the application of direct current voltage 2.5 V (second signal), and a state of open circuit (no electric power-applied state) was given for about 7 seconds. During this period, the change of the electrode pair as the application object for applying the direct current voltage (namely, change from the electrode 4 and the electrode 5 to the electrode 6 and the electrode 7) was performed. At the point in time at which 7 seconds elapsed in the open circuit state, the direct current voltage for measuring the glucose value (200 mV: corresponding to the first signal having the first value) was continuously applied for about 5 seconds. The response current (first electric response value) was measured at the point in time of 14 seconds after the start of the measurement.

Table 1 illustrates results of the measurement of the second electric response values (response currents) of the respective biological samples in which the glucose value was constant (336 mg/dl) and the Hct value was 0%, 20%, 42%, and 70%. The measured value of the second electric response value is the measured value (3 seconds value) obtained at the point in time at which 3 seconds elapse after the start of the measurement as referred to in the measuring method of (2) described above (at the point in time at which the application of the second signal continues for 2.5 seconds).

**[Table 1]**

| **Hct LINEARITY (3 second value)** | | | | |
|---|---|---|---|---|
| **Hct. (%)** | **70** | **42** | **20** | **0** |
| **Current (*µ*A)** | **23.29** | **42.95** | **58.73** | **71.06** |

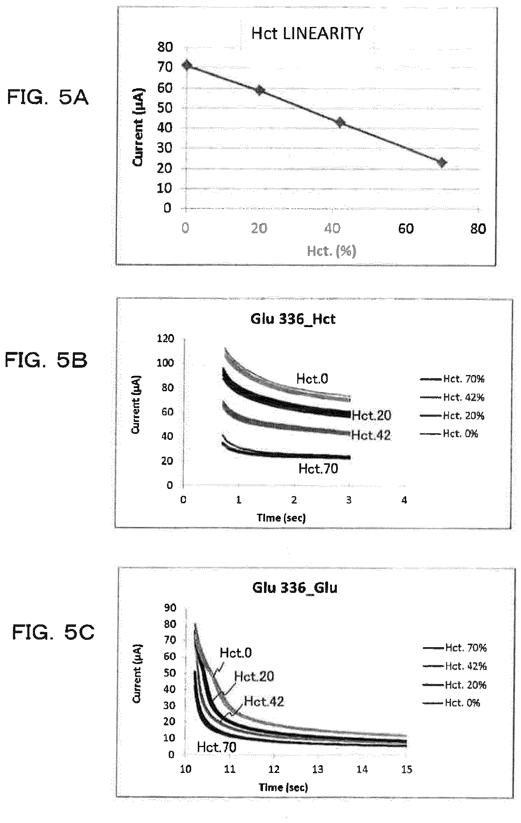

FIG. 5A illustrates a graph illustrating the linearity of the Hct value (Hct linearity) prepared based on the results of the measurement illustrated in Table 1. FIG. 5B illustrates the time-dependent change of the second electric response value measured by using the specimen group 2 (having the constant glucose value).

FIG. 5C illustrates the time-dependent change of the first electric response value measured by using the specimen group 2 (having the constant glucose value).

The graph illustrated in FIG. 5A illustrates the correlation (corresponding relationship) between the second electric response value and the Hct value (linearity of the calibration curve of the Hct value) obtained when the glucose value is constant. As illustrated in FIG. 5A, it has been revealed that the correlation between the second electric response value and the Hct value exhibits the linear form, which can be preferably used as the calibration curve.

Substantially equivalent waveforms were observed as illustrated in FIG. 5B and FIG. 5C, although the larger the concentration concerning the Hct value is, the smaller the response value is. As illustrated in FIG. 5B, the second electric response value, which is obtained when about 2 seconds elapse after the start of the measurement, is substantially constant irrelevant to (irrespective of) the difference in the Hct value (in the range of 0 to 70%). It has been revealed that the stable second electric response value can be obtained even when the reagent is not provided for both of the working electrode and the counter electrode.

Further, as illustrated in FIG. 5C, the first electric response value exhibits the time-dependent change depending on the Hct value, but the first electric response value is the constant value approximately at the point in time exceeding 13 seconds. Accordingly, it is appreciated that the first electric response value, which becomes the constant value after the elapse of the given time, can be measured even when the measurement of glucose is performed following the measurement of the Hct value.

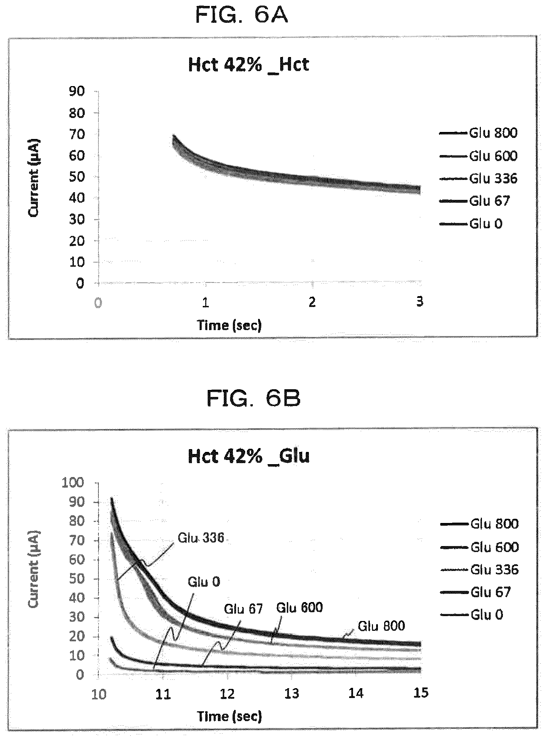

FIG. 6A illustrates the time-dependent change of the second electric response value measured by using the specimen group 1 in which the Hct value is constant (42%). FIG. 6B illustrates the time-dependent change of the first electric response value measured by using the specimen group 1 in which the Hct value is constant (42%) .

As illustrated in FIG. 6A, the time-dependent change of the second electric response value, in which the time-dependent change is approximately equivalent, was observed irrelevant to (irrespective of) the difference in the glucose value (in the range of 0 to 800 mg/dl). As illustrated in FIG. 6B, the lower the glucose value is, the lower the first electric response value is. However, the first electric response value, which was substantially constant approximately after the point in time exceeding 13 seconds, was observed. In this way, it has been revealed that the preferred measurement of glucose is performed even when the glucose value is measured following the measurement of the Hct value. The structures and the arrangements explained in the embodiments can be appropriately combined with each other.

According to the embodiments, it is possible to accurately measure a target substance contained in a biological sample.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to an illustration of the superiority and inferiority of the invention. Although the embodiments of the present invention have been described in detail, it should be understood that the various changes, substitutions, and alterations could be made hereto without departing from the scope of the invention.

## Claims

1. A measuring method for measuring a target substance contained in a sample by using an analysis tool including a flow passage for the sample and a plurality of electrodes arranged in the flow passage, the plurality of electrodes including a first electrode which is provided with a reagent including an enzyme, a second electrode and a third electrode each of which is not provided with the reagent, and a fourth electrode which is different from the first electrode, the second electrode, and the third electrode, the measuring method comprising:
applying a first signal, which is a direct current signal, having a first value to the sample in the flow passage by using the first electrode as a working electrode and using the fourth electrode as a counter electrode;
measuring a first electric response value of the sample to the first signal;
applying continuously for a given time a second signal, which is a direct current signal, having a second value higher than the first value to the sample in the flow passage by using the second electrode and the third electrode as the working electrode and the counter electrode;
measuring a second electric response value of the sample within the given time the second signal, the second electric response value indicating amount of electric charge generated by electrolysis of water in the sample; and
correcting a value obtained from the first electric response value based on the second electric response value.

2. A measuring method for measuring a target substance contained in a sample by using an analysis tool including a flow passage for the sample and a plurality of electrodes arranged in the flow passage, the plurality of electrodes including a first electrode which is provided with a reagent including an enzyme and a second electrode and a third electrode each of which is not provided with the reagent the measuring method comprising:
applying a first signal, which is a direct current signal, having a first value to the sample in the flow passage by using a first electrode as a working electrode and using one of a second electrode and a third electrode as a counter electrode;
measuring a first electric response value of the sample to the first signal;
applying continuously for a given time a second signal, which is a direct current signal, having a second value higher than the first value to the sample in the flow passage by using the second electrode and the third electrode as the working electrode and the counter electrode;
measuring a second electric response value of the sample within the given time to the second signal, the second electric response value indicating amount of electric charge generated by electrolysis of water in the sample; and
correcting a value obtained from the first electric response value based on the second electric response value.

3. The measuring method according to claim 1, wherein the fourth electrode, which is provided with the reagent, is used as the counter electrode in the step of applying the first signal to the sample.

4. The measuring method according to any preceding claim, wherein the second signal is a direct current voltage and the second value ranges from 1V to 7V.

5. The measuring method according to any preceding claim, wherein the sample is blood.

6. The measuring method according to any preceding claim, wherein the target substance is glucose.

7. The measuring method according to any preceding claim, wherein the second electric response value is a value which indicates hematocrit.

8. The measuring method according to claim 7, wherein the first electric response is a value which indicates glucose before being corrected by the value which indicates hematocrit.

9. A measuring apparatus for measuring a target substance contained in a sample by using an analysis tool including a flow passage for the sample and a plurality of electrodes arranged in the flow passage, the plurality of electrodes including a first electrode which is provided with a reagent including an enzyme, a second electrode and a third electrode each of which is not provided with the reagent, and a fourth electrode which is different from the first electrode, the second electrode, and the third electrode, the measuring apparatus comprising:
a first measuring unit configured to apply a first signal, which is a direct current signal, having a first value to the sample in the flow passage by using the first electrode as a working electrode and using the fourth electrode as a counter electrode, and configured to measure a first electric response value of the sample depending on the first signal;
a second measuring unit configured to apply continuously for a given time a second signal, which is a direct current signal, having a second value higher than the first value to the sample in the flow passage by using the second electrode and the third electrode as the working electrode and the counter electrode, and configured to measure a second electric response value of the sample within the given time to the second signal, the second electric response value indicating amount of electric charge generated by electrolysis of water in the sample; and
a correcting unit configured to correct a value obtained from the first electric response value based on the second electric response value.

10. A measuring apparatus for measuring a target substance contained in a sample by using an analysis tool including a flow passage for the sample and a plurality of electrodes arranged in the flow passage, the plurality of electrodes including a first electrode which is provided with a reagent including an enzyme, and a second electrode and a third electrode each of which is not provided with the reagent, the measuring apparatus comprising:
a first measuring unit configured to apply a first signal, which is a direct current signal, having a first value to the sample in the flow passage by using the first electrode as a working electrode and using one of the second electrode and the third electrode as a counter electrode, and configured to measure a first electric response value of the sample depending on the first signal;
a second measuring unit configured to apply continuously for a given time a second signal, which is a direct current signal, having a second value higher than the first value to the sample in the flow passage by using the second electrode and the third electrode as the working electrode and the counter electrode, and configured to measure a second electric response value of the sample within the given time to the second signal, the second electric response value indicating amount of electric charge generated by electrolysis of water in the sample; and
a correcting unit configured to correct a value obtained from the first electric response value based on the second electric response value.
